# EUROPEAN PATENT SPECIFICATION

(11) **EP 1 962 598 B1**
(45) Date of publication and mention of the grant of the patent: **26.11.2014**
(21) Application number: 06845919.7
(22) Date of filing: 19.12.2006
(51) Int. Cl.: A01N 43/04, A61K 31/495, A61K 47/02, A61K 47/10

(54) **HOMOGENEOUS PASTE FORMULATIONS**
HOMOGENE PASTENFORMULIERUNGEN
FORMULATIONS DE PATES HOMOGENES

(30) Priority: 20.12.2005 US 314387
(43) Date of publication of application: 03.09.2008
(73) Proprietor: Merial Limited, Duluth, GA 30096 (US)
(72) Inventor: FREEHAUF, Keith, Stockton, NJ 08559 (US); MOADDEB, Maryam, Collegeville, PA 19426 (US)
(74) Representative: Clyde-Watson, Zöe
(86) International application number: PCT/US2006/048672
(87) International publication number: WO 2007/075827

(56) References cited:
- US-A1- 2003 236 203
- US-B2- 6 787 342

## Description

### FIELD OF THE INVENTION

This invention provides for oral homogeneous veterinary pastes which are used in treating, controlling and preventing of endo- and ectoparasite infections in warm-blooded animals, such as birds, horses and household pets. This invention further provides for a process of preparing these veterinary pastes and for a method for increasing the bioavailability of the anthelmintic agents contained in the paste in the warm-blooded animal. The inventive oral homogeneous anthelmintic pastes comprise a first anthelmintic agent, for example, praziquantel and/or pyrantel, and at least one macrolide anthelmintic compound, a solvent, which dissolves both the first anthelmintic agent and the macrolide anthelmintic compound, and a thickening agent. The inventive oral homogeneous pastes achieve a better bioavailability of the two active anthelmintic agents in the animal than when the two actives are in suspension and not dissolved.

### BACKGROUND OF THE INVENTION

Therapeutic agents are administered to animals by a variety of routes. These routes include, for example, oral ingestion, topical application or parental administration. The particular route selected by the practitioner depends upon factors such as the physiochemical properties of the pharmaceutical or therapeutic agent, the condition of the host, and economic factors.

For example, one method of formulating a therapeutic agent for oral, topical, dermal or subdermal administration is to formulate the therapeutic agent as a paste or as and injectable formulation and reference is made to US application Ser. No. 09/504,741, filed February 16, 2000, now U.S. Patent 6,787,342, entitled **IMPROVED PASTE FORMULATIONS** or to Ser. No. 09/346,905, filed July 2, 1999, now U.S. Patent 6,239,112; Ser. No. 09/112,690, filed July 9, 1999 now U.S. Patent 5,958,888 and Ser. No 09/152,775, filed September 14,1998, now U.S. Patent 6,174,540, entitled **LONG ACTING INJECTABLE FORMULATIONS CONTAINING HYDROGENATED CASTOR OIL.** The disclosure of these patent applications as well as the references cited therein and the references cited herein as well as the references cited in the references are expressly incorporated by reference. Other methods include placing the therapeutic agent in a solid or liquid matrix for oral delivery.

An important area in veterinary science in the control of endo- and ectoparasites in warm-blooded animals, such as equine animals and domestic pets. Infections of parasites, including cestodes and nematodes, commonly occur in animals such as horse, donkeys, mules, zebras, dogs, cats. Various classes anthelmintic agents have been developed in the art to control these infections; see, e.g., U.S, Patents 3,993,682 and 4,032,655, which disclose phenylguanidines as anthelmintic agents. Further, the art recognizes that it is advantageous to administer combinations of two or more different classes of anthelmintic agents in order to improve the spectrum of activity; see, e.g., product disclosure for RM® Parasiticide-10, an anthelmintic paste comprising febantel and praziquantel.

Macrolide anthelmintic compounds are known in the art for treating endo- and ectoparasite infections in warm-blooded animals. Compounds that belong to this class of agents include the avermectin and milbemycin series of compounds. These compounds are potent antiparasitic agents against a wide range of internal and external parasites. Avermectins and milbemycins share the same common 16-membered macrocyclic lactone ring; however, milbemycins do not possess the disaccharide substituent on the 13-position of the lactone ring. In addition to treating parasitic insects, such as flies, avermectins and milbemycins are used to treat endoparasites, e.g., round worm infections, in warm-blooded animals.

The avermectin and milbemycin series of compounds either are natural products or are semi-synthetic derivatives. The natural product avermectins are disclosed in U.S. Patent 4,310,519 to Albers-Schonberg, et al.*,* and the 22,23-dihydro avermectin compounds are disclosed in Chabala, et al., U.S. Patent 4,199,569. For a general discussion of avermectins, which include a discussion of their uses in humans and animals, see "Ivermectin and Abamectin," W.C. Campbell, ed., Springer-Verlag, New York (1989). Naturally occurring milbemycins are described in Aoki et al., U.S. Patent 3,950,360 as well as in the various references cited in "The Merck Index" 12th ed., S. Budavari, Ed., Merck & Co., Inc. Whitehouse Station, New Jersey (1996). Semisynthetic derivatives of these classes of compounds are well known in the art and are described, for example, in U.S. Patent 5,077,308, U.S. Patent 4,859,657, U.S. Patent 4,963,582, U.S. Patent 4,855,317, U.S. Patent 4,871,719, U.S. Patent 4,874,749, U.S. Patent 4,427,663, U.S. Patent 4,310,519, U.S. Patent 4,199,569, U.S. Patent 5,055,596, U.S. Patent 4,973,711, U.S. Patent 4,978,677, and U.S. Patent 4,920,148. All these documents are herein incorporated by reference.

Avermectins and milbemycins are ineffective against cestodes, such as tapeworms, which also are a common parasite in warm-blooded animals (see, U.S. Patent 6,207,179). Of particular importance in the industry is the treatment of equine tapeworms, in general, and *Anoplacephala perfoliata,* in particular (see, e.g., U.S. Patent 6,207,179 or U.S. Patent 5,824,653). In order to treat cestode (and trematode) infections in warm-blooded animals, it is know, to administer 2-acyl-4-oxo-pyrazino-isoquinoline derivatives to the animal (see, e.g., U.S. 4,001,441, herein incorporated by reference). A compound of this class that is often used to treat cestode and nematode infections is praziquantel, which has the following structure:

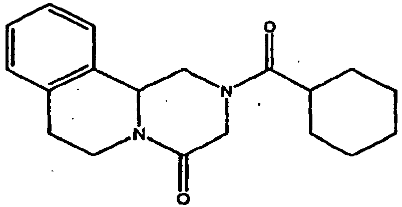

As mentioned above, often it is beneficial to administer a formulation that contains a combination of two or more anthelmintics, which possess different activity, in order to obtain a composition with a broad spectrum of activity. Further, the combination allows the user to administer one formulation instead of two or more different formulations to the animal. Formulations which administer a combination of two or more anthelmintics are know in the art. These formulations may be in the form of solutions, suspensions, pastes, drenches or pour-on formulations (see, e.g., U.S. Patent 6,165,987 to Harvey or U.S. Patent 6,340,672 to Mihalik). For example, U.S. Patent 4,468,390 to Kitano and U.S. Patent 5,824,653 to Beuvry et al. describe anthelmintic compositions for treating nematode and cestode infections in animals, such as horses, that comprise an avermectin or a milbemycin and an isoquinoline compounds, such as praziquantel, to the animal. In these formulations, the avermectin or milbemycin compound and the isoquinoline compound are not dissolved in a solvent, which is then dispersed in a semi-solid matrix. Similarly, U.S. Patent 6,207,179 to Mihalik describes anthelmintic paste formulations wherein the avermectin or milbemycin is dissolved in a non-aqueous liquid and pyrantel or morantel, compounds which are in the same class as praziquantel, but are said in the art to be far less effective as praziquantel, are suspended in the liquid. These prior patents do not describe a formulation wherein the both the praziquantel and the avermectin or milbemycin are dissolved in a solvent and then dispersed in a carrier matrix. U.S. Patent 6,165,987 describes anthelmintic formulations containing praziquantel and at least one avermectin or milbemycin dissolved in an ester or ester-like compounds, such as glycerol formal, benzyl alcohol and N-methyl-2- pyrrolidone, which may be liquids, pastes or drenches; the amount of praziquantel administered to the animal is always at a dose of more that 2.0 mg per kg of body weight U.S. Patent 6,165,987 provides for pastes which require the presence of two solvents, one for the praziquantel and one for the macrolide compound.

Citation or identification of any document in this application is not an admission that such document is available as prior art to the present invention.

### SUMMARY OF THE INVENTION

The present invention provides for a stable paste formulation for a wide range of veterinary and pharmaceutical products. The present invention also provides for an improved process to make the inventive paste products.

The present invention provides for a pharmaceutical or veterinary paste formulation comprising:
(a) an effective amount of a therapeutic agent,
(b) a fumed silica, wherein the fumed silica is colloidal silicon dioxide in a concentration of 5% w/w in the pharmaceutical or veterinary paste formulation,
(c) a viscosity modifier, wherein the viscosity modifier is PEG 400
(d) an absorbent, wherein the absorbent is magnesium carbonate.

The present invention provides for a method for preparing a pharmaceutical or veterinary paste formulation of the invention, said method comprising:
dissolving or dispersing the therapeutic agent into a carrier by mixing,
adding the fumed silica and the absorbent to the carrier containing the dissolved therapeutic agent,
mixing the fumed silica, absorbent and carrier containing the dissolved therapeutic agent at low shear,
maintaining the temperature at about 25°C until the silica and absorbent is dispersed in the carrier and
adding the viscosity modifier to the intermediate with mixing to produce a uniform pharmaceutical or veterinary paste formulation.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of' and "consists essentially of' have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings, in which:
FIG. 1 depicts leverage plots of variables and whole model and
FIG. 2 depicts a prediction profiler.

### DETAILED DESCRIPTION

The present invention provides for a stable paste formulation for a wide range of veterinary and pharmaceutical products. The present invention also provides for an improved process to make the inventive paste products.

The present invention provides for a pharmaceutical or veterinary paste formulation comprising:
(a) An effective amount of a therapeutic agent,
(b) A fumed silica, wherein the fumed silica is colloidal silicon dioxide in a concentration of 5% w/w in the pharmaceutical or veterinary paste formulation,
(c) A viscosity modifier, wherein the viscosity modifier if PEG 400,
(d) An absorbent, wherein the absorbent is magnesium carbonate.

The present invention provides for a method for preparing a pharmaceutical or veterinary paste formulation of the invention, said method comprising:
dissolving or dispersing the therapeutic agent into a carrier by mixing,
adding the fumed silica and the absorbent to the carrier containing the dissolved therapeutic agent,
mixing the fumed silica, absorbent and carrier containing the dissolved therapeutic agent at low shear,
maintaining the temperature at about 25°C until the silica and absorbent is dispersed in the carrier and
adding the viscosity modifier to the intermediate with mixing to produce a uniform pharmaceutical or veterinary paste formulation.

In an advantageous embodiment, the fumed silica is colloidal silicon dioxide CAB-O-SIL (Cabot, TD11), at a concentration of 5% w/w, the viscosity modifier is PEG 400 and the absorbent is advantageously a light magnesium carbonate.

The therapeutic agents which are used in the inventive formulations are those which are known to the practitioner as agents which may be formulated as pastes. Classes of therapeutic agents contemplated by the inventive formulations include insecticides, acaricides, parasiticides, growth enhancers, oil-soluble, nonsteroidal anti-inflammatory drugs (NSAIDS), proton pump inhibitors and antibacterial compounds. Specific classes of compounds which fall within these classes include, for example, avermectins, milbemycins, nodulisporic acid and its derivatives, estrogens, progestins, androgens, substituted pyridylmethyl derivatives, phenylpyrazoles, COX-2 inhibitors, 2-(2-benzimidazolyl)-pyrimidines derivatives, depsipeptides (such as emodepside) and macrolide antibiotics.

The present description also provides for an oral homogeneous anthelmintic veterinary paste, for the treating, controlling and preventing of endo-and ectoparasite infections in warm-blooded animal, which comprises an anthelmintic agent, such a praziquantel, and/or pyrantel and, as a second agent, at least one macrolide anthelmintic agent, a solvent which dissolves both the first anthelmintic agent and the macrolide anthelmintic agent, and a thickening agent.

More specifically, this description provides for an oral homogeneous veterinary paste consisting essentially of praziquantel and/or pyrantel and at least one macrolide anthelmintic compound, a solvent, which dissolves both the praziquantel and/or pyrantel and the macrolide anthelmintic compound, and at least one thickening agent. Preferred are oral homogeneous veterinary pastes consisting essentially of praziquantel and/or pyrantel and at least one macrolide anthelmintic compound, a solvent, which dissolves both the praziquantel and/or pyrantel and the macrolide anthelmintic compound, at least one thickening agent, and at least one viscosity modifier. Another embodiment of the invention is an oral veterinary composition consisting essentially of the inventive oral homogeneous veterinary pastes and an opacifier.

The described oral homogeneous veterinary pastes provide for the combination of at least two different anthelmintic agents, one of which is a macrolide anthelmintic compound. The classes of compounds encompassed by the first agent are well known to practitioners in this art. These compounds include, in addition to praziquantel and its related compounds, anthelmintic agents such as pyrantel (see, U.S. Patent 3,502,661 for a description of pyrantel and its related compounds).

The description provides for an oral homogeneous veterinary paste consisting essentially of praziquantel and/or pyrantel and at least one macrolide anthelmintic compound, a solvent, which dissolves both the praziquantel and/or pyrantel and the macrolide anthelmintic compound, at least one thickening agent, and at least one viscosity modifier. In a preferred embodiment, the macrolide anthelmintic compound is selected from the group consisting of doramectin, abamectin, moxidectin, selamectin and ivermectin; the solvent is glycerol formal, propylene glycol, n-methylpyrrolidone, or dimethyl sulfoxide; the thickening agent is selected from the group consisting of a cellulose, a starch, monothioglycerol, polymers or copolymers of polyvinylpyrrolidone, polymers and copolymers of (meth)acrylate, and a natural gum; and the viscosity modifier is selected from the group consisting of vegetable oils, or hydrogenated vegetable oils. In a preferred embodiment, the thickening agent is hydroxypropylcellulose, xanthum gum or hydroxyethyl starch and the viscosity modifier is hydrogenated castor oil, com oil or olive oil.

The macrolide anthelmintic compounds contemplated in this description are also well known to a practitioner of this area. These compounds include avermectins and milbemycins, some of which are discussed above. Non-limiting examples of compounds belonging to this class are represented by the following structure: 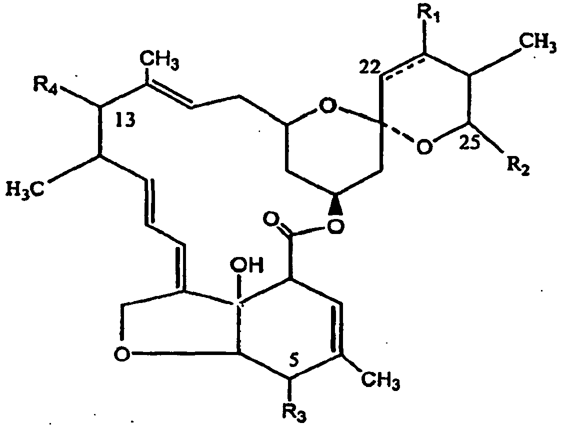 where the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms or cycloalkyl of from 3 to 8 carbon atoms;
R₃ is hydroxy, methoxy or = NOR₅ where R₅ is hydrogen or lower alkyl; and
R₄ is hydrogen, hydroxy or 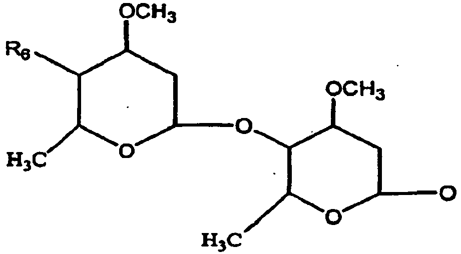 where R₆ is hydroxy, amino, mono-or di-lower alkylamino or lower alkanoylamino.

The preferred compounds are avermectin Bla/Blb (abamectin), 22,23-dihydro avennectin Bla/Blb (ivermectin) and the 4"-acetylamino-5-ketoximino derivative of avermectin Bla/Blb. Both abamectin and ivermectin are approved as broad spectrum antiparasitic agents. The structures of abamectin and ivermectin are as follows: 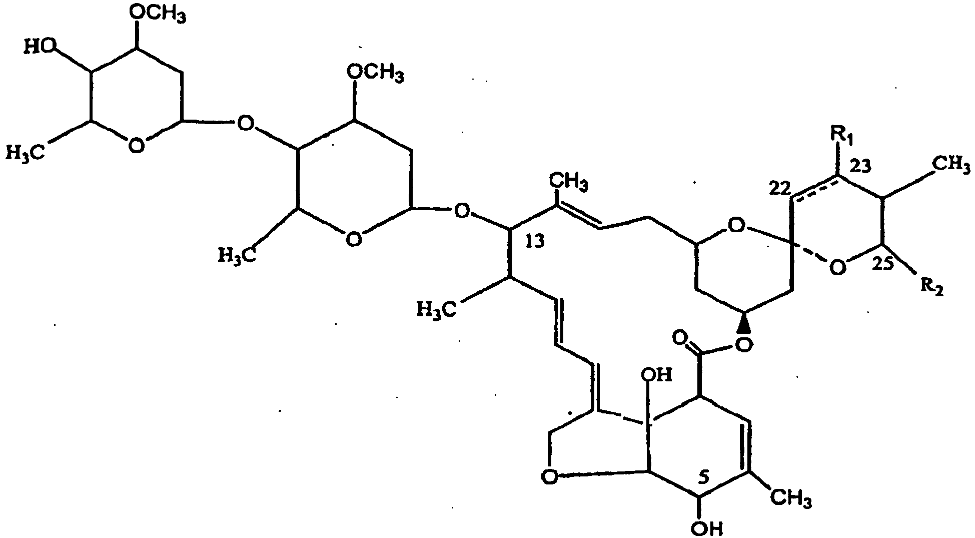 wherein for abamectin the broken line represents a double bond and R₁ is not present and for ivermectin the double bond represents a single bond and R₁ is hydrogen; and R₂ is isopropyl or sec-butyl.

The 4"-acetyl amino-5-ketoximino derivatives of avermectin Bla/Blb has the following structural formula: 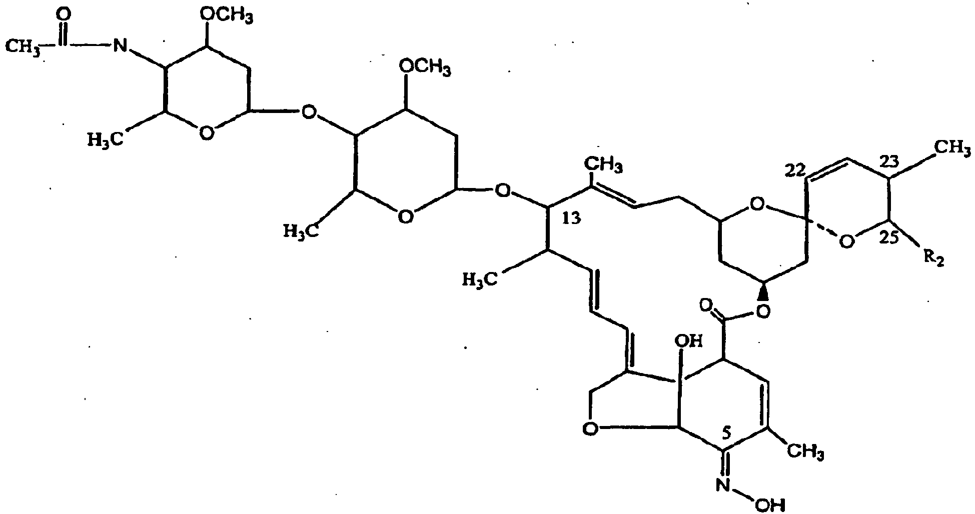 where R₂ is isopropyl or sec-butyl.

The avermectin products are generally prepared as a mixture of at least 80% of the compound where R₂ is sec-butyl and no more than 20% of the compound where R₂ is isopropyl.

Other preferred avermectins, include ememectin, epinomectin and doramectin. Doramectin is disclosed in U.S. Patent 5,089,490 and EP 214 738. This compound has the following structure: 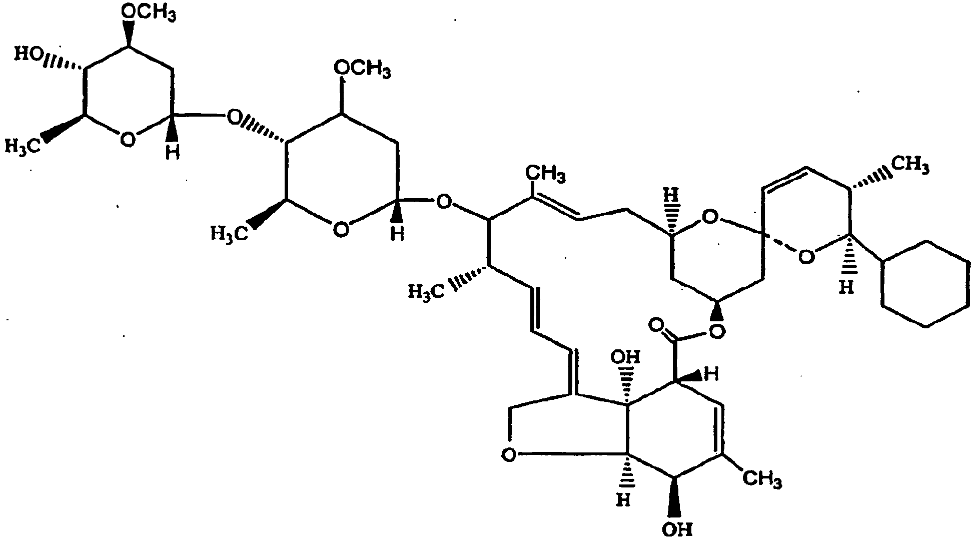 In the present formulations, ivermectin is especially preferred.

A representative structure for a milbemycin is that for milbemycin α₁:

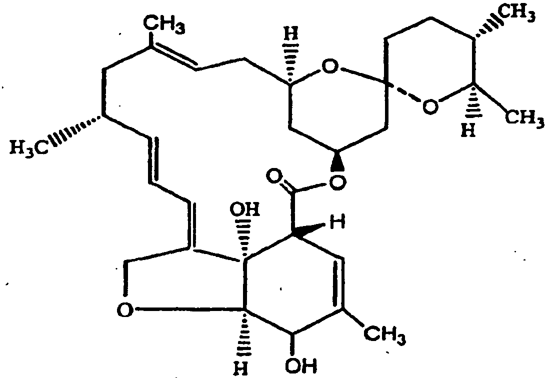

An especially preferred milbemycin is moxidectin, whose structure is as follows: 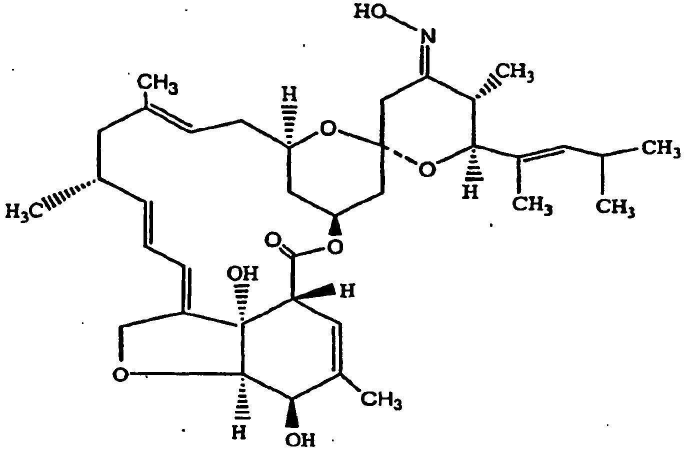 The compound is disclosed in U.S. Patent No. 5,089,490.

The monosaccharide avermectin derivatives are also preferred especially where an oxime substitution is present on the 5-position of the lactone ring. Such compounds are described, for example, in EP 667,054. Selamectin is an especially preferred compound of this class of derivatives.

This application contemplates all pharmaceutically or veterinary acceptable acid or base salts forms of the anthelmintic compounds, where applicable. The term "acid" contemplates all pharmaceutically or veterinary acceptable inorganic or organic acids. Inorganic acids include mineral acids such as hydrohalic acids, such as hydrobromic and hydrochloric acids, sulfuric acids, phosphoric acids and nitric acids. Organic acids include all pharmaceutically or veterinary acceptable aliphatic, alicyclic and aromatic carboxylic acids, dicarboxylic acids tricarboxylic acids and fatty acids. Preferred acids are straight chain or branched, saturated or unsaturated C₁-C₂₀ aliphatic carboxylic acids, which are optionally substituted by halogen or by hydroxyl groups, or C₆-C₁₂ aromatic carboxylic acids. Examples of such acids are carbonic acid, formic acid, fumaric acid, acetic acid, propionic acid, isopropionic acid, valeric acid, α-hydroxy acids, such as glycolic acid and lactic acid, chloroacetic acid, benzoic acid, methane sulfonic acid, and salicylic acid. Examples of dicarboxylic acids include oxalic acid, malic acid, succinic acid, tataric acid and maleic acid. An example of a tricarboxylic acid is citric acid. Fatty acids include all pharmaceutically or veterinary acceptable saturated or unsaturated aliphatic or aromatic carboxylic acids having 4 to 24 carbon atoms. Examples include butyric acid, isobutyric acid, sec-butyric acid, lauric acid, palmitic acid, stearic acid, oleic acid, linoleic acid, linolenic acid, and phenylsteric acid. Other acids include gluconic acid, glycoheptonic acid and lactobionic acid.

The term "base" contemplates all pharmaceutically or veterinary acceptable inorganic or organic bases. Such bases include, for example, the alkali metal and alkaline earth metal salts, such as the lithium, sodium, potassium, magnesium or calcium salts. Organic bases include the common hydrocarbyl and heterocyclic amine salts, which include, for example, the morpholine and piperidine salts.

The ester and amide derivatives of these compounds, where applicable, are also contemplated. Specific compounds which belong to this class of macrolide antiparasitic agents are well known to the practitioner of this art.

The solvents provided for in the described homogeneous pastes are those polar solvent that will dissolve both the first anthelmintic agent and the macrolide anthelmintic compound. These solvents include, for example, glycerol formal, 1-methylpyrrolidone (NMP), dimethyl sulfoxide (DMSO). Glycerol formal exists in two isomeric forms, the α,α'-form and the α,β-form. These forms are reproduced below:

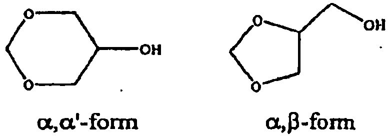

The thickeners contemplated by this description are well known to a practitioner of this art. Compounds which function as thickeners include, for example, celluloses, starches, natural gums, monothioglycerol, synthetic polymers, such as polymers and copolymers of polyvinylpyrrolidone or (meth)acrylates, etc. Especially preferred thickeners are hydroxypropylcellulose, xanthum gum and hydroxyethyl starch. Thickeners may be present in amounts of from about 3% to about 30%.

Opacifiers may be added to absorb and/or reflect certain light and/or energy of certain wavelengths and may thus enhance the stability of the formulations. Opacifiers include, for example, zinc oxide or titanium dioxide and may be present in amounts from about 0.5 to 2.5%. Titanium dioxide is especially preferred. These compounds are well known to practitioners of this art.

Additionally, the inventive formulations may contain other inert ingredients such as antioxidants, preservatives, or pH stabilizers. These compounds are well known in the formulation art. Antioxidant such as an alpha tocopheral, ascorbic acid, ascrobyl palmitate, fumeric acid, malic acid, sodium ascorbate, sodium metabisulfate, n-propyl gallate, BHA (butylated hydroxy anisole), BHT (butylated hydroxy toluene) monothioglycerol and the like, may be added to the present formulation. The antioxidants are generally added to the formulation in amounts of from about 0.01 to about 2.0%, based upon total weight of the formulation, with about 0.05 to about 1.0% being especially preferred. Preservatives, such as the parabens (methylparaben and/or propylparaben), are suitably used in the formulation in amounts ranging from about 0.01 to about 2.0%, with about 0.05 to about 1.0% being especially preferred. Other preservatives include benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, and the like. Preferred ranges for these compounds include from about 0.01 to about 5%.

Colorants may be added to the inventive formulations. Colorants contemplated by the present invention are those commonly known in the art. Specific colorants include, for example, dyes, an aluminum lake, caramel, colorant based upon iron oxide or a mixture of any of the foregoing. Especially preferred are organic dyes and titanium dioxide. Preferred ranges include from about 0.5% to about 25%.

Compounds which stabilize the pH of the formulation are also contemplates. Again, such compounds are well known to a practitioner in the art as well as how to use these compounds. Buffering systems include, for example, systems selected from the group consisting of acetic acid/acetate, malic acid/malate, citric acid/citrate, tataric acid/tartrate, lactic acid/lactate, phosphoric acid/phosphate, glycine/glycimate, tris, glutamic acid/glutamates and sodium carbonate. Preferred ranges for pH include from about 4 to about 6.5.

The inventive pastes may be administered to warm-blooded animals. Warm-blooded animals include, for example, all ruminants, equines, canines, felines and avians. Especially preferred are birds, cattle, sheep, pigs, dogs, cats, horses and the like. The amount of each of anthelmintic compounds is well known to a practitioner of this art. Preferred amounts of prazequantel include, for example, from about 0.5 mg/kg to about 7.5 mg/kg of animal body weight, with a range of about 0.5 mg/kg to about 2 mg/kg or 2.5 mg/kg of body weight being especially preferred. A most especially preferred amount is about 1.0 mg/kg of animal body weight. Preferred ranges for the anthelmintic macrolide compounds include, for example about 0.01 to about 200 mg/kg of animal body weight, with the ranges of about 0.1 to about 50 mg/kg and from about 1 to about 30 mg/kg being especially preferred.

The described oral homogeneous pastes may be prepared, for example, by a process which comprises:
-- dissolving the at least two different anthelmintic agents, e.g., praziquantel or pyrantel and macrolide anthelmintic compound or compounds, into the solvent; and
- adding the thickening agent or agents and stirring until a homogeneous paste is formed.
More preferred processes comprise:
-- dissolving the at least two different anthelmintic agents, e.g., praziquantel or pyrantel, and macrolide anthelmintic compound or compounds, and thickening agent or agents into the solvent and forming a thickened solution;
-- cooling the thickened solution to a temperature below about 35° C
-- adding the viscosity modifier agent and stirring until a homogeneous paste is formed or
-- dissolving the at least two different anthelmintic agents, e.g., praziquantel or pyrantel, and macrolide anthelmintic compound or compounds, the thickening agent or agents and least one compound selected from the group consisting of an antioxidant, a colorant, a pH stabilizer and/or a preservative into the solvent and forming a thickened solution;
-- cooling the solution to a temperature below about 35° C; and
-- adding the viscosity modifying agent or agents and stirring until a homogeneous paste is formed.

A preferred process to prepare the described oral veterinary compositions comprises:
--dissolving the at least two different anthelmintic agents, e.g., parzequantel or pyrantel, and at least one macrolide anthelmintic compound or compounds and the thickening agent or agents into the solvent and forming a thickened solution;
--adding the opacifier to the thickened solution and mixing until the opacifier is evenly dispersed;
--cooling the thickened solution with the evenly dispersed opacifier to a temperature below about 35° C;
--adding the viscosity modifier and stirring until the oral veterinary composition is formed.

The inventive oral veterinary formulations may be used to treat a number of ecto-and endoparasite infections. The determining of a treatment protocol for an infection of a specific parasite or parasites would be well within the skill level of a practitioner of the veterinary art. This invention further provides for a method to increase the bioavailability of the at least two different anthelmintic agents in the animal.

The invention will now be further described by way of the following non-limiting example

### EXAMPLES

### Reference example, 1: Oral Veterinary Homogeneous Paste

An oral veterinary homogeneous paste, which had the following ingredients:

| **INGREDIENTS** | **AMOUNT (% w/w)** |
|---|---|
| Praziquantel | 7.75 |
| Ivermectin | 1.55 |
| Butylated hydroxyanisole (BHA) | 0.02 |
| Sunset Yellow (FD&C Yellow No. 6) | 0.04 |
| Titanium dioxide | 2.0 |
| Hydroxypropylcellulose (HPC) | 6.0 |
| Hydrogenated castor oil | 4.0 |
| Stabilized glycerol formal | QS AD 100 |

was prepared by the following process:
1. Add some or all of the stabilized glycerol formal to a mixture followed by the addition of the praziquantel, ivermectin and BHA. The ingredients are mixed until they are dissolved in the stabilized glycerol formal.
2. Add sunset yellow to the solution and mix until dissolved.
3. Add titanium dioxide to the solution and mix until completely dispersed.
4. Add the remainder of glycerol formal, if necessary.
5. Add HPC to the solution and mix the solution until a homogeneous, viscous solution is obtained.
6. Cool the solution to a temperature below 35°C.
7. Once the solution is cooled to a temperature below 35°C, add the hydrogenated castor oil, while mixing, until all the hydrogenated castor oil is mixed into the solution; the temperature of the solution is maintained below 35°C.
8. Once the hydrogenated castor oil has been added, increase the agitation speed of the mixer while heating the mixture.
9. Mix until the product is a paste.

### Example 2: Use of Statistically Designed Experiments for Formulation Optimization of a Semisolid

A lower percentage of active was desired in a paste formulation with a resulting increase in the percentage of solvent. After the formulation change was implemented, observations of paste separation raised concerns over the manufacturing process, physical stability and end user elegance. To address these concerns, lab scale optimization studies of the formulation were undertaken in an effort to eliminate or reduce separation and the results are summarized below.

It is believed that the structure of the paste formed from hydrogen bonding between the colloidal silicon dioxide and the polyethylene glycol (see, e.g., Raghavan et al., Langmuir 2000, 16, 7920-7930). The main objective of this example was to test the effect of the following factors on the physical stability (phase separation) of the paste formulation:
(a) amount of colloidal silicon dioxide (Cab-O-Sil) at either 4 or 5%
(b) type of magnesium carbonate (light or heavy)
(c) type of polyethylene glycol (PEG 300 or PEG 400)
(d) shear used during manufacture (high or low shear)
(e) temperature of paste during manufacture (25 C or 45 C)

A statistical experimental design was used to provide main and interaction effects of the factors. The goal of the experimental design was to find the optimum parameters which would result in a product with minimal or no liquid separation.

The experimental design was based on the two continuous factors (amount of colloidal silicon dioxide and temperature) at two levels and three categorical factors (type of magnesium carbonate, type of PEG and the intensity of shear) at two levels. Table 1 describes the factors and levels that were evaluated in the experiments.

The paste produced from each experimental run was subjected to accelerated stress by centrifugation to force the separation of liquid from the paste. Since the goal of the experimental design was to minimize the separation of liquid under normal storage conditions, the weight of the separated liquid was considered the response for each experimental run. The accelerated stress condition is necessary, because at normal storage conditions the paste takes too much time to separate. It is assumed that the comparative stability of different pastes under this accelerated test condition will be the same as that at the normal storage conditions. Only one replicate for each experimental run was evaluated and it was assumed that the variability between runs is negligible. Using JMP® SAS software, a fractional factorial screening design of 16 experimental runs with randomized run order was generated (Table 1). This design included all the main effects and second order interaction effects without confounding.

A stock solution of active in triacetin was prepared. To 15 g of triacetin-drug stock solution, titanium dioxide, magnesium carbonate (light or heavy), and 4% w/w or 5% w/w colloidal silicon dioxide was added using a Lightnin mixer with an appropriate size impeller. The mixer speed was set at either 300 rpm (low shear) or 800 rpm (high shear) and the beaker was set in a circulating water bath maintained at either 25 C or 45 C according to the requirements of the experimental run. As a final step, remaining triacetin and viscosity modifier (PEG 300 or PEG 400) were added to the beaker while mixing so as to make a 50 g paste. Paste was removed from the beaker and approximately 8 grams was centrifuged at 15,000 rpm for 15 minutes and the resulting supernatant liquid was weighed.

The weight of the liquid from each experimental run is included alongside the different parameters for each run. The average weight of liquid is the response factor. The above data was fitted to a multiple regression model which included main effects and 2^{nd} order interactions.

**Table 1: Design of Experiments and their results**

| **Run No.** | **Colloidal SiO₂, % w/w** | **MgCO₃ type** | **Viscosity Modifier type** | **Shear Intensity** | **Temp. (C)** | **Weight of liquid, g** | | |
|---|---|---|---|---|---|---|---|---|
| | | | | | | **Tube 1** | **Tube 2** | **Avg.** |
| 1 | 4 | Heavy | PEG400 | Low | 45 | 3.0726 | 2.9635 | 3.018 |
| 2 | 5 | Heavy | PEG300 | Low | 45 | 2.4013 | 2.2292 | 2.315 |
| 3 | 4 | Heavy | PEG400 | High | 25 | 3.1701 | 3.0601 | 3.115 |
| 4 | 5 | Light | PEG300 | Low | 25 | 1.8128 | 1.7567 | 1.785 |
| 5 | 5 | Heavy | PEG300 | High | 25 | 1.7167 | 1.6695 | 1.693 |
| 6 | 5 | Heavy | PEG400 | Low | 25 | 1.6302 | 1.7079 | 1.669 |
| 7 | 5 | Light | PEG400 | High | 25 | 1.8434 | 1.8817 | 1.863 |
| 8 | 4 | Heavy | PEG300 | Low | 25 | 2.8748 | 2.8450 | 2.860 |
| 9 | 5 | Heavy | PEG400 | High | 45 | 1.8680 | 1.8740 | 1.871 |
| 10 | 4 | Light | PEG400 | Low | 25 | 2.9016 | 2.8292 | 2.865 |
| 11 | 5 | Light | PEG300 | High | 45 | 1.7862 | 1.8679 | 1.827 |
| 12 | 4 | Light | PEG300 | High | 25 | 2.7648 | 2.8402 | 2.803 |
| 13 | 4 | Heavy | PEG300 | High | 45 | 2.8487 | 2.6880 | 2.768 |
| 14 | 5 | Light | PEG400 | Low | 45 | 1.9213 | 1.9697 | 1.946 |
| 15 | 4 | Light | PEG400 | High | 45 | 3.0419 | 3.0740 | 3.058 |
| 16 | 4 | Light | PEG300 | Low | 45 | 3.0446 | 2.8130 | 2.929 |

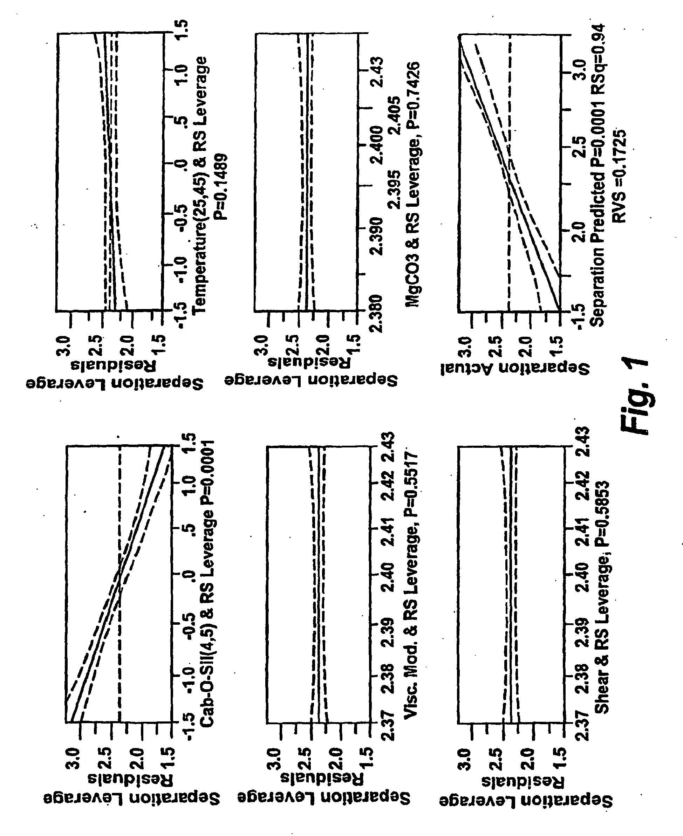

The leverage plots are provided for each of the variable factors as well as the whole model as shown in FIG. 1. The strength of the effect is shown by the slope of the central fit line. The greater the slope (positive or negative), the greater the effect that variable has on the paste separation. The distance from each point to the central fit line is what the error would be if the variable is taken out of the model. Confidence curves on the graph show whether an effect is significant or not. If the 95% confidence curves cross from the horizontal reference line, then the effect is significant; if the curves do not cross, then it is not significant.

From the plots of FIG. 1, it is evident that only the amount of colloidal dioxide in the formulation is very significant and the effect of temperature is marginally significant. Other variables, i.e., light or heavy MgCO₃, PEG 300 or PEG 400, or high or low shear are not significant. The interaction effects for these other variables are not shown.

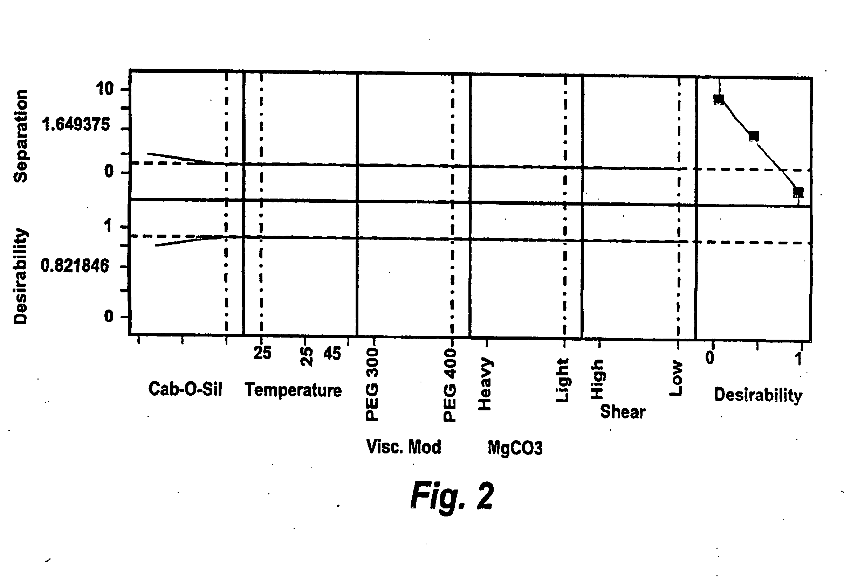

The prediction profiling shown in FIG. 2 displays predict ion traces for each X variable. The importance of a variable can be assessed to some extent by the steepness of the prediction line. It appears that the amount of colloidal silicon dioxide has maximum effect on the separation of liquid. The predictor profile is a useful tool in calculating the different scenarios that are of interest from the predicted model.

The desirability function is set to minimize the response factor. In other words, the desirability function when maximized calculates the parameters for the variables such that the predicted formulation produces least amount of phase separation. The following table provides the parameters for the variables when the desirability function is maximized. Predicted formulation for the least amount of phase separation is shown in Table 2.

**Table 2: Predicted formulation**

| **Variable Factor** | **Desired Value** |
|---|---|
| Colloidal silicon dioxide | 5% w/w |
| MgCO₃ | Light |
| Viscosity Modifier | PEG 400 |
| Shear | Low |
| Temperature | 25 C |

It was evidence from the experiments performed and the analysis of the data that increasing the concentration of colloidal silicon dioxide from 4 to 5% will provide the least phase separation. The conclusion was reached by an analysis of data from an experimental design that utilized the least number of experimental runs. This same analysis also predicted a formulation which will have the least separation based on the variables evaluated.

## Claims

1. A pharmaceutical or veterinary paste formulation comprising:
(a) an effective amount of a therapeutic agent,
(b) a fumed silica, wherein the fumed silica is colloidal silicon dioxide in a concentration of 5 % w/w in the pharmaceutical or veterinary paste formulation,
(c) a viscosity modifier, wherein the viscosity modifier is PEG 400,
(d) an absorbent, wherein the absorbent is magnesium carbonate.

2. The pharmaceutical or veterinary paste of claim 1, wherein the magnesium carbonate is a light magnesium carbonate.

3. The pharmaceutical or veterinary paste of claim 1 wherein the therapeutic agent is selected from the group consisting of avermectins, milbemycins, nordulisporic acid and its derivatives, estrogens, progestins, androgens, substituted pyridyl methyl derivatives, phenylpyrazoles, COX-2 inhibitors or 2-(2-benzimidazolyl)-pyrimidine derivatives.

4. The pharmaceutical or veterinary paste of claim 1 wherein the therapeutic agent comprises praziquantel and ivermectin.

5. The pharmaceutical or veterinary paste of claim 4 wherein the therapeutic agent comprises dissolved praziquantel and dissolved ivermectin.

6. The pharmaceutical or veterinary paste of claim 1 further comprising a carrier.

7. The pharmaceutical or veterinary paste of claim 6 wherein the carrier is a triacetin, a monoglyceride, a diglyceride, or a triglyceride.

8. The pharmaceutical or veterinary paste of claim 7 wherein the carrier is a triacetin.

9. The pharmaceutical or veterinary paste of claim 1 further comprising a colorant, stabilizer, surfactant or preservative.

10. A method for preparing a pharmaceutical or veterinary paste formulation according to any one of claims 6 to 8, said method comprising:
dissolving or dispersing the therapeutic agent into a carrier by mixing,
adding the fumed silica and the absorbent to the carrier containing the dissolved therapeutic agent,
mixing the fumed silica, absorbent and carrier containing the dissolved therapeutic agent at low shear,
maintaining the temperature at about 25°C until the silica and absorbent is dispersed in the carrier and
adding the viscosity modifier to the intermediate with mixing to produce a uniform pharmaceutical or veterinary paste formulation.

11. The method of claim 10 wherein the mixing at low shear is at 300 rpm.

## Patentansprüche

1. Eine pharmazeutische oder veterinärmedizinische Pastenformulierang, umfassend:
(a) eine wirksame Menge eines therapeutischen Mittels,
(b) eine pyrogene Kieselsäure, wobei die pyrogene Kieselsäure kolloidales Siliziumdioxid in einer Konzentration von 5 % w/w in der pharmazeutischen oder veterinärmedizinischen Pastenformulierun.g ist,
(c) ein Viskositätsmodifizierungsmittel, wobei das Viskositätsmodifizierungsmittel PEG 400 ist,
(d) ein Absorptionsmittel, wobei das Absorptionsmittel Magnesiumcarbonat ist.

2. Die pharmazeutische oder veterinärmedizinische Paste nach Anspruch 1, wobei das Magnesiumcarbonat ein leichtes Magnesiumcarbonat ist.

3. Die pharmazeutische oder veterinärmedizinische Paste nach Anspruch 1, wobei das therapeutische Mittel aus der Gruppe, bestehend aus Avermectinen, Milbemycinen, Nordulisporinsäure und deren Derivaten, Östrogenen, Progestinen, Androgenen, substituierten Pyridylmethylderivaten, Phenylpyrazolen, COX-2-Inhibitoren oder 2-(2-Benzimidazolyl)-pyrimidinderivaten, ausgewählt ist.

4. Die pharmazeutische oder veterinärmedizinische Paste nach Anspruch 1, wobei der therapeutische Wirkstoff Praziquantel und Ivermectin umfasst.

5. Die pharmazeutische oder veterinärmedizinische Paste nach Anspruch 4, wobei das therapeutische Mittel gelöstes Praziquantel und gelöstes Ivermectin umfasst.

6. Die pharmazeutische oder veterinärmedizinische Paste nach Anspruch 1, weiter umfassend einen Träger.

7. Die pharmazeutische oder veterinärmedizinische Paste nach Anspruch 6, wobei der Träger ein Triacetin, ein Monoglycerid, ein Diglycerid oder ein Triglycerid ist.

8. Die pharmazeutische oder veterinärmedizinische Paste nach Anspruch 7, wobei der Träger ein Triacetin ist.

9. Die pharmazeutische oder veterinärmedizinische Paste nach Anspruch 1, weiter umfassend einen Farbstoff, einen Stabilisator, ein oberflächenaktives Mittel oder ein Konservierungsmittel.

10. Ein Verfahren zur Herstellung einer pharmazeutischen oder veterinärmedizinischen Pastenformulierung nach einem der Ansprüche 6 bis 8, wobei das Verfahren umfasst:
Auflösen oder Dispergieren des therapeutischen Mittels in einem Träger durch Mischen,
Zugeben der pyrogenen Kieselsäure und des Absorptionsmittels zu dem Träger, der das gelöste therapeutische Mittel enthält,
Mischen der pyrogenen Kieselsäure, des Absorptionsmittels und des Trägers, der das gelöste therapeutische Mittel enthält, bei niedriger Scherung,
Halten der Temperatur bei etwa 25 °C, bis die Kieselsäure und das Absorptionsmittel in dem Träger dispergiert sind, und
Zugeben des Viskositätsmodifizierungsmittels zu dem Zwischenprodukt unter Mischen, um eine gleichmäßige pharmazeutische oder veterinärmedizinische Pastenfornaulierung herzustellen.

11. Das Verfahren nach Anspruch 10, wobei das Mischen bei niedriger Scherung bei 300 Umdrehungen pro Minute erfolgt.

## Revendications

1. Formulation de pâte pharmaceutique ou vétérinaire comprenant:
(a) une quantité efficace d'un agent thérapeutique,
(b) une silice fumée, où la silice fumée est du dioxyde de silicium colloïdal en une concentration de 5 % p/p dans la formulation de pâte pharmaceutique ou vétérinaire,
(c) un modificateur de viscosité, où le modificateur de viscosité est le PEG 400,
(d) un absorbant, où l'absorbant est le carbonate de magnésium.

2. Pâte pharmaceutique ou vétérinaire selon la revendication 1 où le carbonate de magnésium est un carbonate de magnésium léger.

3. Pâte pharmaceutique ou vétérinaire selon la revendication 1 où l'agent thérapeutique est choisi dans le groupe consistant en les avermectines, les milbémycines, l'acide nordulisporique et ses dérivés, les estrogènes, les progestines, les androgènes, les dérivés de pyridylméthyle substitués, les phénylpyrazoles, les inhibiteurs de COX-2 et les dérivés de 2-(2-benzimidazolyl)-pyrimidine.

4. Pâte pharmaceutique ou vétérinaire selon la revendication 1 où l'agent thérapeutique comprend du praziquantel et de l'ivermectine.

5. Pâte pharmaceutique ou vétérinaire selon la revendication 4 où l'agent thérapeutique comprend du praziquantel dissous et de l'ivermectine dissoute.

6. Pâte pharmaceutique ou vétérinaire selon la revendication 1 comprenant en outre un vecteur.

7. Pâte pharmaceutique ou vétérinaire selon la revendication 6 où le vecteur est une triacétine, un monoglycéride, un diglycéride ou un triglycéride.

8. Pâte pharmaceutique ou vétérinaire selon la revendication 7 où le vecteur est une triacétine.

9. Pâte pharmaceutique ou vétérinaire selon la revendication 1 comprenant en outre un colorant, un stabilisant, un tensioactif ou un conservateur.

10. Procédé pour préparer une formulation de pâte pharmaceutique ou vétérinaire selon l'une quelconque des revendications 6 à 8, ledit procédé comprenant:
la dissolution ou la dispersion de l'agent thérapeutique dans un vecteur par mélange,
l'addition de la silice fumée et de l'absorbant au vecteur contenant l'agent thérapeutique dissous,
le mélange de la silice fumée, de l'absorbant et du vecteur contenant l'agent thérapeutique dissous à faible cisaillement,
le maintien de la température à environ 25°C jusqu'à ce que la silice et l'absorbant soient dispersés dans le vecteur et
l'addition du modificateur de viscosité à l'intermédiaire avec mélange pour produire une formulation de pâte pharmaceutique ou vétérinaire uniforme.

11. Procédé selon la revendication 10 où le mélange à faible cisaillement est à 300 tr/min.
